# EUROPEAN PATENT SPECIFICATION

(11) **EP 1 759 711 B1**
(45) Date of publication and mention of the grant of the patent: **11.06.2008**
(21) Application number: 05019082.6
(22) Date of filing: 02.09.2005
(51) Int. Cl.: A61K 47/02, A61K 47/36

(54) **Chitosan-silicon dioxide coprecipitate and use as excipient in solid dosage forms**
Chitosan-Siliziumdioxid-Co-Präzipitat und Verwendung als Hilfsstoff in festen Dosierungsformen
Co-précipité de chitosan-dioxyde de silicium et utilisation comme excipient dans des formes posologiques solides

(43) Date of publication of application: 07.03.2007
(73) Proprietor: The Jordanian Pharmaceutical Manufacturing Co., 11710 Naor (JO)
(72) Inventor: Badwan, Adnan Dr., 11185 Amman (JO); Al-Remawi, Mayyas Dr., 13713 Russiefa (JO)
(74) Representative: Winkler, Andreas Fritz Ernst

(56) References cited:
- US-B1- 6 344 488
- SINGLA A K ET AL: "CHITOSAN: SOME PHARMACEUTICAL AND BIOLOGICAL ASPECTS - AN UPDATE" JOURNAL OF PHARMACY AND PHARMACOLOGY, LONDON, GB, vol. 53, no. 8, August 2001 (2001-08), pages 1047-1067, XP008019188 ISSN: 0022-3573

## Description

### Field of invention

The present invention relates to chitosan as an excipient with preferable physical properties.

### Background of the invention

The present invention relates to a novel crystalline silicated chitosan excipient for the preparation of direct compressible readily flowable chitosan particles.

Chitosan is a very abundant polymer obtained by alkaline deacetylation of chitin (a polymer made of acetylglucosamine units). Chitin is present in the exoskeletons of crustaceans, the cuticles of insects and cell walls of most fungi. Chitosan is a heteropolymer containing both glucosamine and acetylglucosamine units. The presence of amine groups explains its cationic behavior in acidic solutions and its affinity to anionic substances and metals. 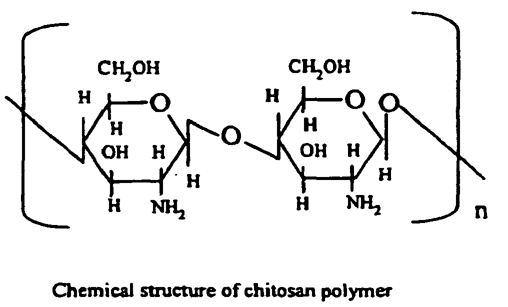 [E. Guibal, Heterogenous catalysis on chitosan-based materials: a review. Prog. Polym. Sci., 30 (2005) 71-109]
Chitosan as it is a natural substance and highly available in nature, is inexpensive, nontoxic, biodegradable, and biocompatible when compared with other polymers. Chitosan is considered to be non-digestible by humans when taken via the oral route; this is due to lack of chitosanases, which are present in some bacteria. [K. Oungbho; and B. Muller. Chitosan Sponges as Sustained Release Drug Carriers. Int.J. Pharm.156, pp 229-237, 1997]

Pharmaceutical uses of chitosan are very numerous. The scientific and medical literature lists hundreds of industrial, medical and dietary applications for chitosan. These include protection of sensitive drugs from the deactivating enzymes, preparation of artificial cells, using chitosan to trap hemoglobin, promotion of bone repair, burn dressing and contact lens material. As a drug delivery vehicle, chitosan is considered as the drug carrier for the 21st century. Chitosan has potential as permeation enhancer for hydrophilic macromolecular compounds and peptide drugs. Chitosan has been examined extensively by the pharmaceutical industry for its potential in controlled drug delivery systems. The polymeric cationic character together with its potential reactive groups give chitosan unique properties for utilization in controlled release technology.
[J. Karlsen, and O. Skaugrud. Exicipient Properties of Chitosan. Manufacturing Chemist., 62, pp 18, 1991; I Orienti, K. Aieda, C. Ponti, E. Gianasi, V. Zecchi. Progesterone Loaded Chitosan Microspheres. Effect of triethylene glycol glutarate linked to the chitosan molecule on drug release. S.T.P. Pharm. Sci., 6, pp 424-429, 1996; H. Takeuchi, H. Yamamoto, T. Niwa; T. Hino and Y. Kawashima. Internal absorption of insulin in rats from mucoadhesive chitosan-coated liposomes. Pharm. Res.13, pp 896-901, 1996]

Oral drug delivery systems of chitosan were prepared depending on its physicochemical and pharmaceutical properties:
Chitosan direct compression tablets offered a sustained release of the water-insoluble drug prednisolone [W. Paul, C. Sharma. Chitosan, a drug carrier for the 21st century: a review. STP Pharma Sci.,10, pp 5-22, 2000]
Chitosan is a polymer that has a good mucoadhesion property. Actually, mucoadhesive polymeric systems have generated significant interest over the years as a method to improve the performance of controlled release drug delivery systems. This is because such systems can localize the formulation at the site of interest for local or systemic delivery, optimize contact with the gastrointestinal tissue surface to improve drug permeability and they can easily be combined with auxiliary agents, such as enzyme inhibitors, adhesion and penetration enhancers. [K. Oungbho; and B. Muller. Chitosan Sponges as Sustained Release Drug Carriers. Int.J. Pharm.156, pp 229-237, 1997]
Chitosan beads can be formed when chitosan acidic solution being dropped on a sodium tripolyphosphate solution. These beads were prepared as controlled release drug carrier. Chitosan conjugates with either drugs or other chemical moieties to modify its properties. Chitosan-EDTA was developed as a novel bioadhesive polymer that protects peptide drugs from luminal degradation by aminopeptidase N. The conjugation of indomethacin with chitosan partially substituted with hydrophilic residues; triethylene glycol glutarate and triethylene glycol choline ether glutarate were described. [L. Wan, L. Lim, B. Soh. Drug Release from Chitosan Beads. S.T.P. Pharma Sci, 4, pp 195-200, 1994; C. Aral, J. Akbuga. Alternative Approach to the preparation of Chitosan Beads. Int. J. Pharm.,168, pp 9-15, 1998; Y. Murata Y., T. Maeda, Miyamoto, S. Kawashima. Preparation of Chitosan-reinforced alginate gel beads- effect of chitosan on gel matrix erosion. Int J Pharma. 96, pp 139-145, 1993; A. Bernkop-Schnurch, C. Paikl, C. Valenta. Novel Bioadhesion Chitosan-EDTA Conjugate Protects Leucine Enkeplalin from Degradation by Aminopeptidase N. Pharm. Res., 14, pp 917-922, 1997; I. Orienti I, K. Aieda., E. Gianasi, C. Ponti, V. Zecchi. Chitosan Indomethacin Conjugates. Effect of Different Substituents on the Polysaccharide Molecule on Drug Release. Arch. Pharm. Pharm. Med, Chem. 329, pp 245-250, 1996]
S.T.P. Pharma in Jan-Feb 2000 published a complete thematic issue on "chitosan in drug delivery systems". Also, there are several review articles on chitosan as a drug carrier. [W. Paul, C. Sharma. Chitosan, a drug carrier for the 21st century: a review. STP Pharma Sci.,10, pp 5-22, 2000; F. Olivia, P. Buri., R. Gury. Chitosan: A unique Polysaccharide for Drug Delivery: a review. Drug Dev. Ind.Pharm. 24, pp 979-993, 1998]

The main focus herein is on silicated chitosan as an excipient for solid dosage forms.

In order to prepare a solid dosage form many additives are used, since it is necessary that the material to be compressed into the tablet dosage form possesses certain physical characteristics. Among these, the material to be compressed must be free flowing, must be lubricated, and, most importantly, must possess sufficient cohesiveness to insure that the solid dosage form remains intact after compression.

The simplest method for tablet formation is the direct compression method. However, the use of direct compression is limited in those situations where the drug and/or excipients have a requisite crystalline structure and physical characteristics required for formation of a pharmaceutically acceptable tablet. However, it is well known in the art to include one or more excipients, which make the direct compression method applicable for materials of poor physical properties. For solid dosage forms wherein the material of poor physical properties is to be included in a relatively high dose (e.g., it comprises a substantial portion of the total tablet weight), it is necessary that this material should possess sufficient physical characteristics (e.g., cohesiveness) for the ingredients to be directly compressed.

In case of chitosan products, where chitosan forms the majority of the tablet, chitosan powder must possess good flow and cohesiveness properties. But this is not the situation in most cases. Different formulation methodologies are adopted to improve its flow by the increase in chitosan particle size.

Dry granulation procedures are useful to improve flowability. These procedures may be utilized, where one of the constituents that possess good flow properties is added to the formula. The method includes mixing the ingredients, slugging, dry screening, lubricating and finally compressing the ingredients.

Another method is a wet granulation procedure which may be useful for the enhancement of flowability and includes mixing the powders to be incorporated into the dosage form and, thereafter adding solutions of a binding agent to the mixed powders to obtain granulation. Thereafter, the damp mass is screened, and dried, to obtain granules with a specific particle size distribution.

Also, any other method of particle size enlargement known in the art could be helpful. However, these methods usually cost money, time and labor. An important defect seen in the methods of particle size enlargement is the poor compressibility of the formed tablet. The products that contain a high percentage of chitosan usually suffer from low particle to particle bonding i.e. low cohesiveness. This condition increases the chance of tablet friability. This was observed in elastic recovery studies to elucidate bonding mechanisms developed by the tablet after compression of fine powder and granule of larger particle size of chitosan formulations.
[Formulation of a Universal Controlled Release Hydrophilic Polymer Mixtures Ready for Direct Compression, Jordan University of Science and Technology, May, 2003, thesis submitted in partial fulfilment of the requirement of the degree of Ph.D. in pharmaceutical technology by M. Al-Remawi, supervised by Dr. A. Badwan and Dr. M. Salem]

The method of coprecipitation of chitosan with silica increases the particle size i.e. improves flow properties and surprisingly, increases chitosan compressibility properties.

### Summary of the invention

A chitosan-based excipient having improved flowability, compressibility, whether utilized in direct compression, dry granulation or wet granulation formulations, is disclosed. The excipient is a chitosan silicon dioxide precipitate composition, preferably a coprecipitate of chitosan polymer acidic solution and silicon dioxide colloidal alkaline dispersion, where the optimal composition contains silicon dioxide in the range of 1-75% w/w, and the most preferable concentration is 50% w/w. Chitosan and silicon dioxide are in intimate association with each other. The silicon dioxide has a particle size in the nanometer range to about 100 microns. The most preferable grade is colloidal silicon dioxide.

### Detailed description of the invention

Chitosan is used in sustained release tablet formulations as a release-retarding agent. Also, it is used in immediate release formulations as an active agent for fat binding.

Typically chitosan has a bulk density of about 0.15 g/cc to 0.4 g/cc.

High-density chitosan grades reach up to 0.6 g/cc (2-3 times more than the ordinary chitosan).
[http://www.uchitotech.com/Specifications/specifications.html#CHITOSANTOP].

The density of chitosan is a matter of particle size. The lower particle size chitosan usually has a low bulk density (higher volume to weight) but it has a high compressibility and bad flow properties. On the other hand, the larger particle size chitosan possesses higher density, but lower compressibility and good flow properties.

This is an industrial problem when chitosan is incorporated in large percentage in tablets either as a fat binding therapy or as an excipient in sustained release for high strength tablets. The use of low density chitosan results in a problem of die filling before compression i.e. only small amount of chitosan will fill the die completely and so the tablet produced will be very thin. Also, low-density grades suffer from poor flow properties. This makes a great problem in production upon the use of the high-speed compression machines that requires excellent flowability. However, the use of high density chitosan will solve the problem of die filling, and flow. But the tablets formed will suffer from weak bonding i.e. fragile tablets are formed.

When the novel excipient of the invention utilizes a colloidal silicon dioxide, it has been found that the resultant excipient product surprisingly provides a compressibility, which is substantially improved in preferred embodiments even in comparison to normal commercially available high-density grades of chitosan.

The optimal composition contains silicon dioxide in the range of 1-75% w/w, and the most preferable concentration is 50% w/w.

Both chitosan in the form of a wet mass and silicon dioxide are substantially water-insoluble. Therefore, there is no appreciable dissolution of either ingredient in the aqueous slurry. After a uniform mixture of the ingredients is obtained in a wet mass, the wet mass is dried to provide chitosan-based excipient particles.

According to the present invention the particles comprise as one essential component chitosan.

Chitosan materials for use herein have an average degree of deacetylation of more than 50%, preferably from 75% to about 100% and most preferably from 85% to about 100%.

Chitosan materials which may generally have a wide range of molecular weights are suitable for use in the present invention, typically chitosan materials for use herein have a molecular weight ranging from 1,000 to 10,000,000 gram per mole and more preferably 2,000 to 1,000,000 gram per mole.

Chitosan materials pH depends on the preparation of chitosan. The preferred pH is acidic, typically in the range of 4 to 6. However, basic ranges of pH, typically above 8, may be preferable for the preparation of chitosan primary amine base.

Particularly suitable aminoglycosides for use herein include aminopolysaccharides salts, especially chitosan salts including organic and inorganic salts known in the art.

Particularly suitable modified chitosans for use herein include water-soluble or water-insoluble covalently bonded chitosan derivatives or ionically bonded chitosan derivatives known in the art.

According to the present invention the articles comprise as the other essential component silica.

Silica i.e. silicon dioxide exists in a variety of crystalline forms and amorphous modifications, any of which are suitable for the use herein. In particular, silica as having a high surface area is preferred such as colloidal silica.

According to the present invention the articles typically comprise from 5 to 300gm⁻², more preferably from 10-250 gm⁻², most preferably from 15 to 200 gm⁻² of silica.

Also, any modified or salts of silica such as silicates are also suitable in the present invention.

Optional agents such as fillers, lubricants, fat absorbing materials, pH buffering agents may be included to facilitate and/or improve coprecipitate properties.

Methods of coprecipitation may include any industrial technique known in the art such as spray drying, or freeze drying or simple solution mixing.

Chitosan products are mainly presented in solid dosage forms as sustained release formulations. However, silicated chitosan is suitable for both immediate release and sustained release formulations depending on the formula used as shown in the following examples.

### Examples

### Example 1

### Crystallinity of chitosan silica coprecipitate

To prove the crystallinity two techniques were used: powder x-ray diffraction and infrared spectra measurements.

X-ray diffractometer (Philips PW 1729 X-Ray Generator). The XRD patterns were measured with x-ray diffractometer. Radiations generated from Co K_{α} source and filtered through Ni filters with a wavelength of 1,79025 Å at 40 mA and 35 kV were used. The instrument was operated over the 2θ range of 5-65°.

Infrared were obtained using FTIR 480, Jasco, Japan. Fourier transformation Infra red spectrometer under room air at room temperature and KBr disk. Samples were placed in oven at 105 °C for 3 hrs before doing any measurements to get rid of moisture. Approximately 150 mg of KBr and 5 mg of sample powder were blended with pestle and mortar for 5 min. The sample disk was prepared at a pressure of 9 tons for 2 min.

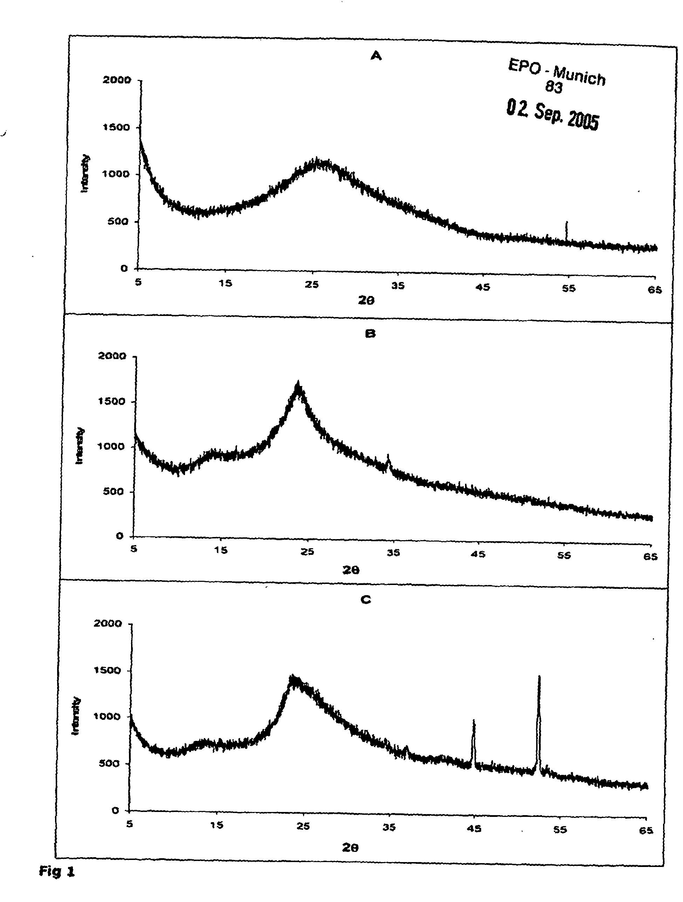

Chitosan and colloidal silica have no sharp x-ray peaks indicating their amorphous nature, as shown in Fig 1. The sharp new two peaks appeared at 2 0 around 45 and 50 in coprecipitated silicated chitosan is an indication of the crystallinity of this modified form of chitosan.

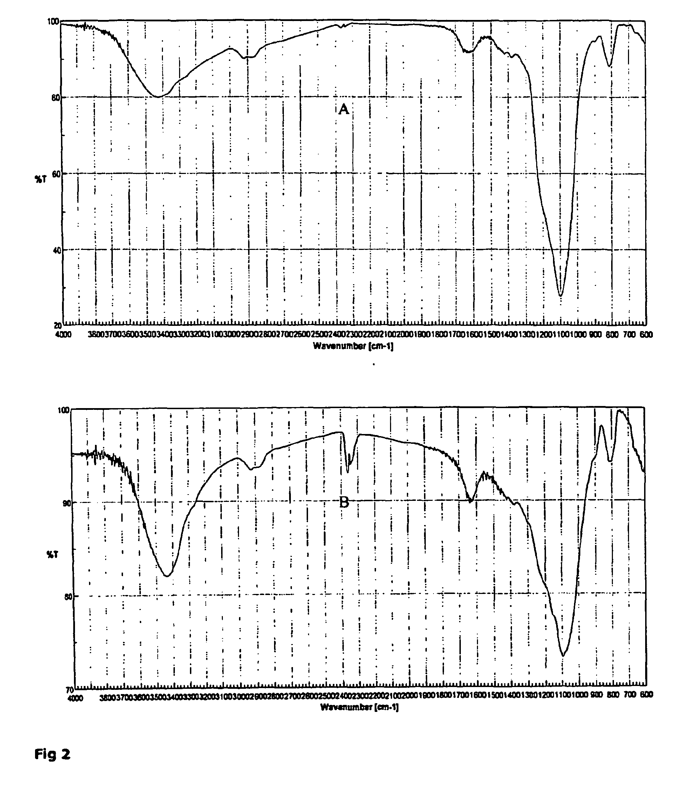

The IR spectra of the physical mixture of chitosan and silica showed a smooth IR spectra with no projections or sharpness, as shown in Fig. 2. The coprecipitated silicated chitosan showed sharp peaks and the presence of shoulders with the major peaks. This indicates that the molecules were present in an ordered crystalline state.

These results indicated that the coprecipitation method resulted in production of crystalline forms of chitosan within the amorphous matrix. This improves its physical properties as shown in the next example.

### Example 2

### Improvement of chitosan physical properties by coprecipitation with silica

Chitosan powder with bulk density (0.19 g/cm3) and colloidal silicone dioxide with bulk density (0.03 g/cm3). This means the two materials are highly fluffy and porous.

Colloidal silica was dispersed in alkaline medium and chitosan in acidic medium. Then, acidic chitosan solution was added with stirring to alkaline colloidal silica. There coprecipitation occur. The pH is monitored to precipitate chitosan polymer completely. The coprecipitate is washed out from salts, dried in oven at 120 °C. Particles were sieved using sieve 0.425 mm.

Tables 1-4 summarize the physical characteristics of silicated chitosan particles.
Chitosan particles without silica have good flow properties and bad compressibility properties as shown in Table 1. While upon coprecipitation of chitosan with silica in a suitable ratio the flow properties and compressibility properties were improved significantly as shown in Tables 2-4.

**Table 1: Summary of physical properties of chitosan precipitated with no silica (CH:silica 100:0).**

| Physical parameter | Value | Unit | Comment | |
|---|---|---|---|---|
| particle size | <0.425 | mm | | |
| bulk density | 0.5 | g/ml | high density | |
| tap density | 0.5 | g/ml | | |
| CI | 8.7 | | Excellent flow | |
| tablet hardness | | | | |

| Punch | wt (gm) | Compression force (kg) | hardness (N) | comment |
|---|---|---|---|---|
| 15 mm circular | 1.0 | 4000.0 | 71.0 | fragile |
| 10 mm circular | 0.5 | 3000.0 | 74.0 | fragile |
| 7 mm circular | 0.2 | 2000.0 | 61.0 | fragile |

**Table 2: Summary of physical properties of 25% w/w chitosan precipitated with 75% w/w silica (CH:silica 75:25)**

| Physical parameter | Value | Unit | Comment | |
|---|---|---|---|---|
| particle size | <0.425 | mm | | |
| bulk density | 0.4 | g/ml | high density | |
| tap density | 0.4 | g/ml | | |
| CI | 14.0 | | Excellent flow | |
| tablet hardness | | | | |

| Punch | wt (gm) | Compression force (kg) | hardness (N) | comment |
|---|---|---|---|---|
| 15 mm circular | 1.0 | 4000.0 | 547.0 | very hard |
| 10 mm circular | 0.5 | 3000.0 | 547.0 | very hard |
| 7 mm circular | 0.2 | 2000.0 | 547.0 | very hard |

**Table 3: Summary of physical properties of 50% w/w chitosan precipitated with 50% w/w silica (CH:silica 50:50)**

| Physical parameter | Value | Unit | Comment | |
|---|---|---|---|---|
| particle size | <0.425 | mm | | |
| bulk density | 0.5 | g/ml | high density | |
| tap density | 0.6 | g/ml | | |
| CI | 10.0 | | Excellent flow | |
| tablet hardness | | | | |

| Punch | wt (gm) | Compression force (kg) | hardness (N) | comment |
|---|---|---|---|---|
| 15 mm circular | 1.0 | 4000.0 | 545.0 | very hard |
| 10 mm circular | 0.5 | 3000.0 | 545.0 | very hard |
| 7 mm circular | 0.2 | 2000.0 | 545.0 | very hard |

**Table 4: Summary of physical properties of 25% w/w chitosan precipitated with 75% w/w silica (CH:silica 25:75)**

| Physical parameter | Value | Unit | Comment | |
|---|---|---|---|---|
| particle size | <0.425 | mm | | |
| bulk density | 0.3 | g/ml | high density | |
| tap density | 0.3 | g/ml | | |
| CI | 16.7 | | Excellent flow | |
| tablet hardness | | | | |

| Punch | wt (gm) | Compression force (kg) | hardness (N) | comment |
|---|---|---|---|---|
| 15 mm circular | 1.0 | 4000.0 | 160.0 | fragile and capping |
| 10 mm circular | 0.5 | 3000.0 | 160.0 | fragile and capping |
| 7 mm circular | 0.2 | 2000.0 | 100.0 | fragile and capping |

### Example 3

### The use of silicated chitosan in sustained release tablet formulations

A sustained release tablet was prepared using silicated chitosan.
The system contains 120 mg pseudoephedrine HCl and release modifying excipient (chitosan or chitosan modified in concurrent with xanthan gum), as shown in Tables 5 and 6. Components of each tablet were geometrically mixed by porcelain mortar and pestle for about 10 minutes before compression. Circular planar tablets were manufactured with a diameter of 10 mm. Compression of powder mixtures by applying a pressure of about 200 MPa for 15 seconds by a hydraulic press.

**Table 5: summarizes the formulae used for the development of pseudoephedrine HCl sustained release product compared to Contac 12 hr caplet non drowsy ®.**

| **Formula #** | **1** | **2** | **3** | **4** | **5** |
|---|---|---|---|---|---|
| **Constituents (mg/tablet)** | | | | | |
| **Pseudoephedrine HCl** | **120** | **120** | **120** | **120** | **120** |
| **Chitosan modified*** | **120** | **120** | **120** | **120** | **120** |
| **Xanthan gum** | **120** | **120** | **120** | **120** | **120** |
| **Total** | **360** | **360** | **360** | **360** | **360** |
| ***CH:silica ratio** | **100:0** | **100:0** | **75:25** | **50:50** | **25:75** |

| | | | | | |
|---|---|---|---|---|---|
| Reference commercial sustained release product is Contac 12 hr caplet non drowsy ® BN 3JE 0897 (Manufactured by GlaxoSmithKline, US patent no. 5,895,663) Formula 1 represents chitosan as received from its source (fine powder). Formula 2 represents chitosan precipitated in same procedure but without using silica (chitosan treated particle size <0.425 mm) Formulas 3-5 represent coprecipitated chitosan and silica at different ratios, particle size <0.425 mm. | | | | | |

**Table 6: summarizes the formulae used for the development of pseudoephedrine HCl sustained release using modified chitosan 50:50.**

| **Formula #** | **6** | **7** |
|---|---|---|
| **Constituents (mg/tablet)** | | |
| **Pseudoephedrine HCl** | **120** | **120** |
| **Chitosan modified*** | **150** | **180** |
| **Xanthan gum** | **120** | **120** |
| **Total** | **390** | **420** |
| ***CH:silica ratio** | **50:50** | **50:50** |

The USP apparatus II (Paddle) was used. The vessels were placed in a water bath regulated to maintain temperature of 37 ± 0.5 oC during the test. A fitted cover was used on the vessel to prevent any evaporation during the test time. All tablets were subjected to 1000 ml. The tablets are subjected to 0.1 M HCl for 1 hr then 0.1 M sodium phosphate buffer pH 7.5 for the rest of the dissolution period. The speed of dissolution test was set at 50 rpm. At specified time intervals, 5-ml aliquots were withdrawn. At each time interval an aliquot equal in volume to the withdrawn sample was replaced to maintain the original volume of dissolution medium.
Samples of dissolution runs were analyzed using a validated UV spectrophotometer method. The details of the analysis conditions are shown in Table 7.

**Table 7: UV spectrophotometer parameters applied for the analysis of pseudoephedrine HCl**

| Scan type | Second derivative |
|---|---|
| Scan rate | 1200 nm/min |
| Maximum peak | 220 nm |
| Smooth | 25 |

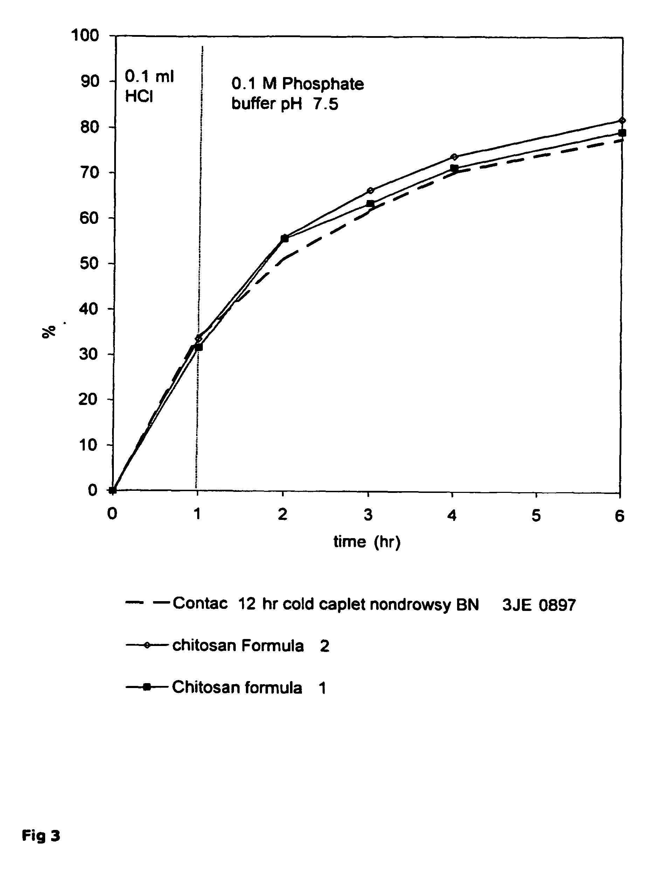

Pseudoephedrine HCl release from the commercial product was compared with the optimal formula (formula 1 determined by previous work. The optimal formula contains chitosan fine powder, as shown in Fig 3. The precipitated chitosan with no silica has a larger particle size (formula 2) compared to chitosan fine powder (formula 1). Nevertheless, it has a similar release behavior i.e. the process of precipitation that lead to size enlargement did not significantly affected the drug release behavior.

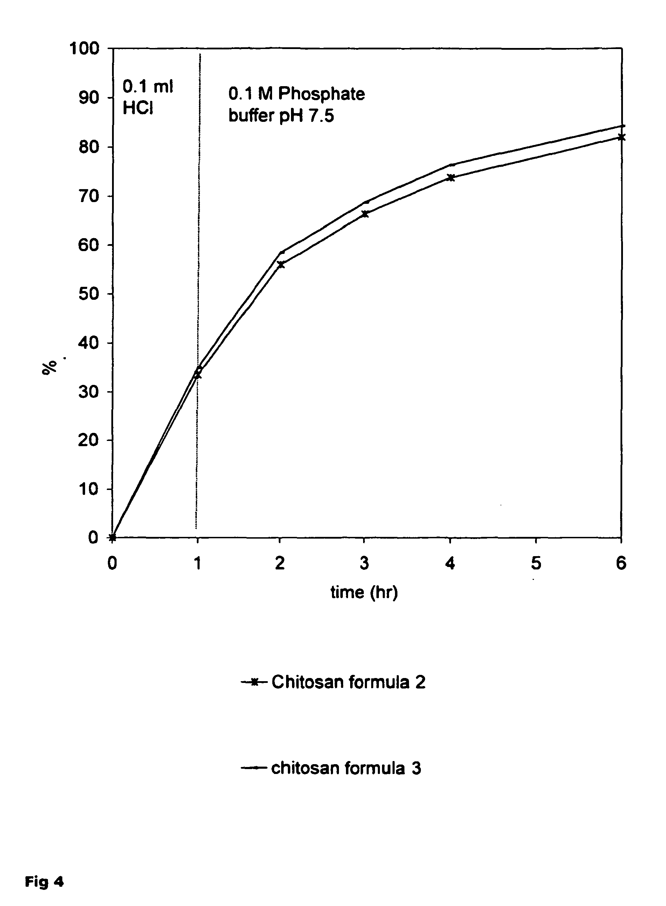

The release profile was also similar to the optimal formula release even when chitosan amount is decreased and replaced by silica as the case with formula 3, Fig. 4. The amount of chitosan was decreased in this formula but the release behavior was not changed. This is due to silica that replaces chitosan at this percentage. An economical advantage of using modified chitosan can be manifested herein. Silica price is about 10 times cheaper than chitosan.

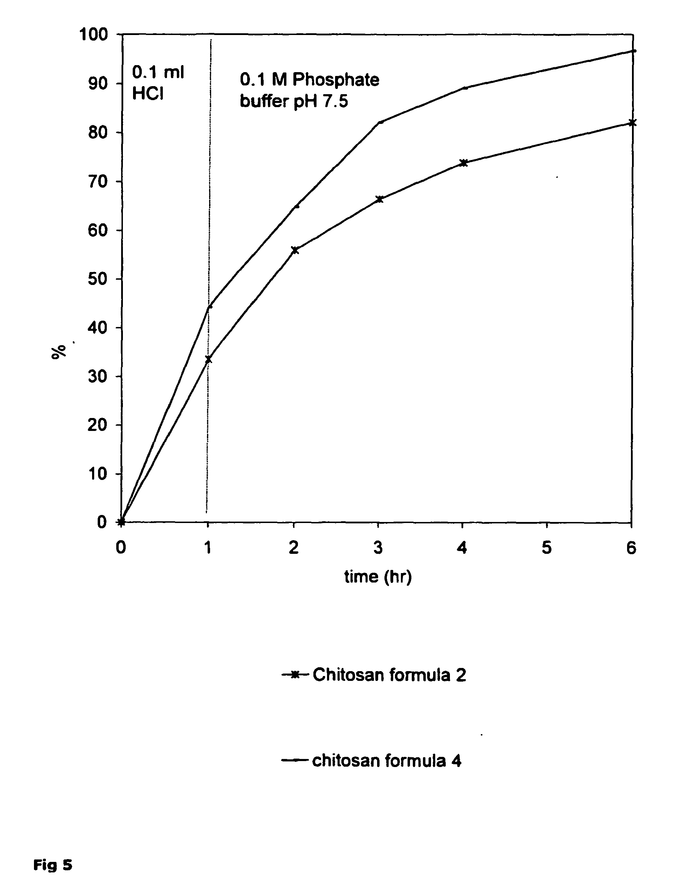

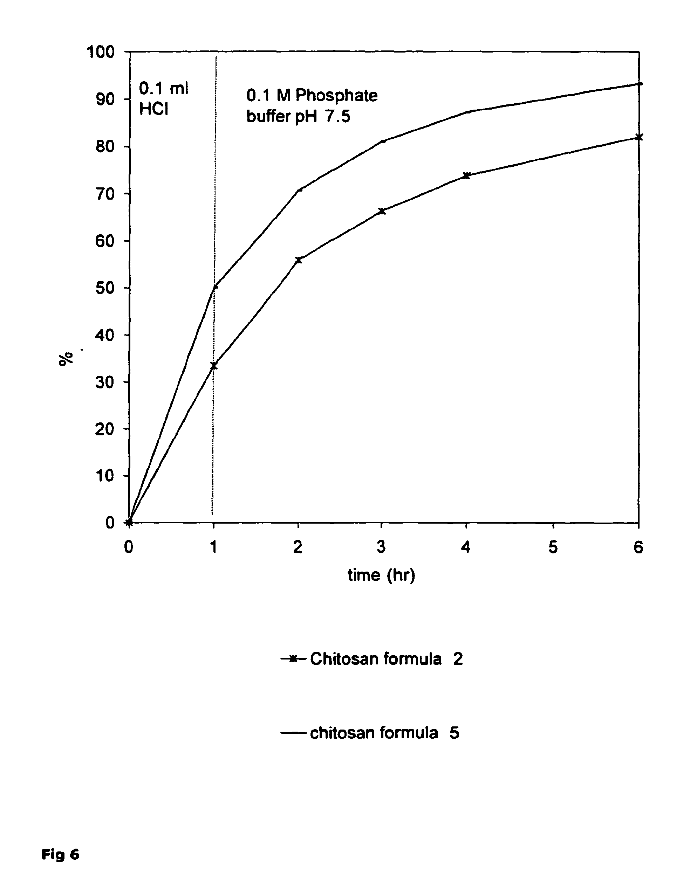

As the amount of silica increased on the expense of chitosan especially (formula 4 and 5) the release retardation power decreased significantly as shown in Figs 5 and 6.

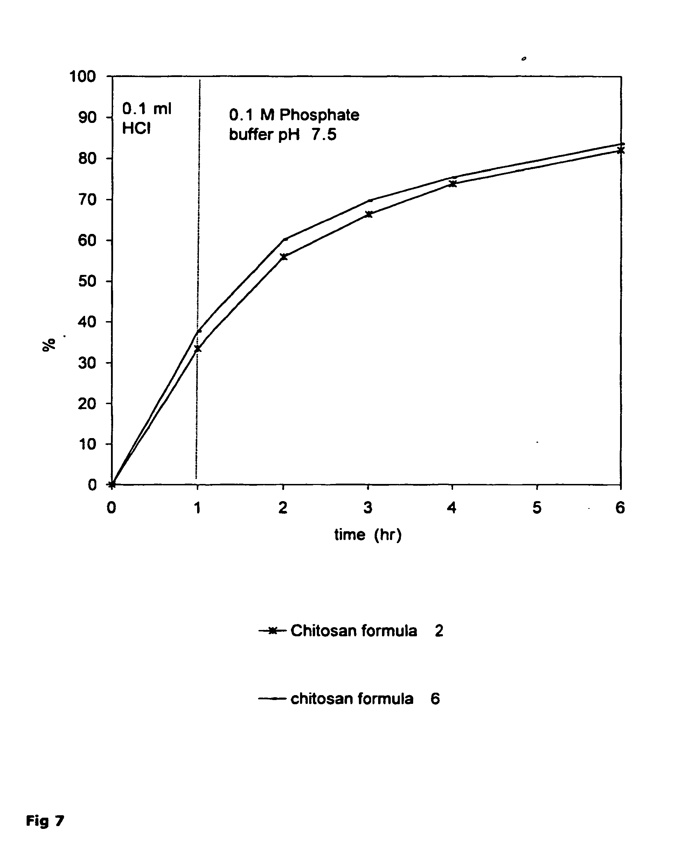

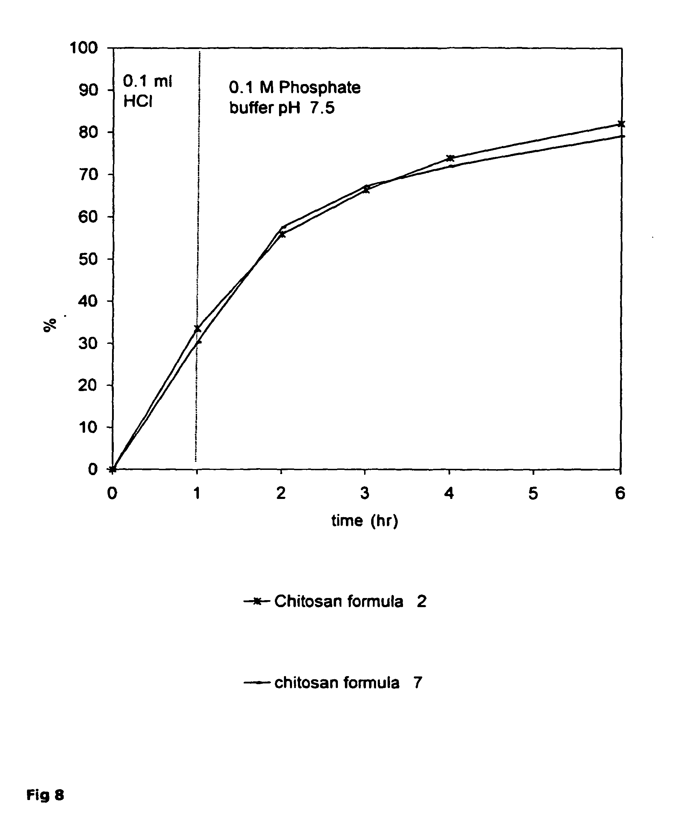

However, this does not mean that the release retardation power got lost. Actually, the release retardation power can be recovered again upon the increase in the amount of chitosan included in the tablet as observed with (formula 6 and 7 ) in Figs 7 and 8.

### Example 4:

### Immediate release tablet was prepared using silicated chitosan.

The system contains 60-mg pseudoephedrine HCl and 60-mg chitosan excipient (chitosan or chitosan modified with silica), as shown in Table 8. Components of each tablet were geometrically mixed by porcelain mortar and pestle for about 10 minutes before compression. Circular planar tablets were manufactured with a diameter of 9 mm. Compression of powder mixtures by applying a pressure of about 200 MPa for 15 seconds by a hydraulic press.

**Table 8: summarizes the formulae used for the development of immediate release chitosan tablet**

| **Formula #** | **8** | **9** |
|---|---|---|
| **Constituents (mg/tablet)** | | |
| **Pseudoephedrine HCl** | **60** | **60** |
| **Chitosan** | **60** | **60** |
| **Total** | **120** | **120** |
| ***CH:silica ratio** | **100:0** | **50:50** |

| | | |
|---|---|---|
| Formula 8 represents chitosan as received from its source (fine powder). Formula 9 represents chitosan precipitated in same procedure but without using chitosan: silica 50:50 (particle size <0.425 mm) | | |

The USP apparatus II (Paddle) was used. The vessels were placed in a water bath regulated to maintain temperature of 37 ± 0.5 °C during the test. A fitted cover was used on the vessel to prevent any evaporation during the test time. All tablets were subjected to 900 ml water. The speed of dissolution test was set at 50 rpm. At specified time intervals, 5-ml aliquots were withdrawn. At each time interval an aliquot equal in volume to the withdrawn sample was replaced to maintain the original volume of dissolution medium. Samples of dissolution runs were analyzed using a validated UV method as mentioned previously in example 3.

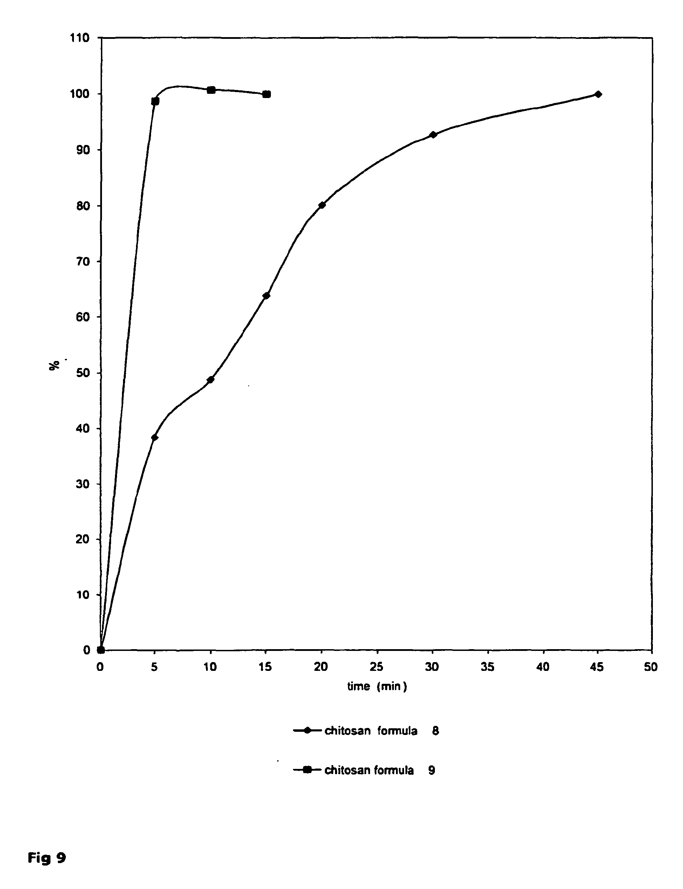

Pseudoephedrine HCl release from chitosan matrix (formula 8) was compared with the silicated chitosan matrix formula (formula 9), as shown in Fig. 9. The silicated chitosan matrix showed a rapid disintegration condition (within 5 minutes) where the entire drug content is released immediately to the dissolution medium, while the ordinary chitosan matrix showed a slow drug release. This release happened due to the drug high solubility in dissolution medium. The differences in drug release suggest that the silicated chitosan absorb more moisture due to the presence of silica that leads to tablet disintegration. Thus, the new silicated chitosan is suitable for immediate release dosage forms.

As a conclusion, besides silica improved the physical properties of chitosan particles it also enhances its drug release retardation power when used with another polymer and it makes chitosan suitable for immediate release dosage forms when used alone or with a disintegrant.

### Figures

Fig 1:Powder X-ray diffraction of A) silica B) chitosan powder C) coprecipitate chitosan:silica 50:50 w/w
Fig 2: IR spectra of A) physical mixture chitosan : silica 50:50 w/w B) coprecipitate chitosan:silica 50:50 w/w
Fig 3: In vitro dissolution profile of pseudoephedrine HCl from sustained release systems of Contac 12 hr ®, chitosan formula I and chitosan formula 2 described in the invention
Fig 4: In vitro dissolution profile of pseudoephedrine HCl from sustained release systems of chitosan formula 2 and formula 3 described in the invention
Fig 5: In vitro dissolution profile ofpseudoephedrine HCl from sustained release systems of chitosan formula 2 and formula 4 described in the invention
Fig 6: In vitro dissolution profile of pseudoephedrine HCl from sustained release systems of chitosan formula 2 and formula 5 described in the invention
Fig 7: In vitro dissolution profile of pseudoephedrine HCl from sustained release systems of chitosan formula 2 and formula 6 described in the invention
Fig 8: In vitro dissolution profile ofpseudoephedrine HCl from sustained release systems of chitosan formula 2 and formula 7 described in the invention
Fig 9: In vitro dissolution profile of pseudoephedrine HCl from immediate release system of chitosan and silicated chitosan (50:50).

## Claims

1. Chitosan silicon dioxide coprecipitate composition.

2. Composition according to claim 1, wherein the content of silicon dioxide is in the range of 1 to 75% w/w.

3. Composition according to claim 2, wherein the content of silicon dioxide is about 50% w/w.

4. Composition according to any of the preceding claims, wherein the silicon dioxide is present in crystalline or amorphous form or as a modified form or salt of silicon dioxide such as silicate.

5. Composition according to claim 4, wherein the silicon dioxide is present in the form of colloidal silicon dioxide.

6. Composition according to any of the preceding claims, wherein the chitosan material has an average degree of deacetylation of more than 50%.

7. Composition according to claim 6, wherein the chitosan material has an average degree of deacetylation of more than 75%.

8. Composition according to claim 7, wherein the chitosan material has an average degree of deacetylation of more than 85%.

9. Composition according to any of the preceding claims, wherein the chitosan material has an average molecular weight ranging from 1.000 to 10.000.000 g/mol.

10. Composition according to claim 9, wherein the chitosan material has an average molecular weight ranging from 2000 to 100.000 g/mole.

11. Composition according to any of the preceding claims, wherein the chitosan material is a chitosan salt with an organic or inorganic acid.

12. Method of production of a chitosan silicon dioxide coprecipitate composition according to any of claims 1 to 11, wherein the method of coprecipitation is selected from spray drying, freeze drying or solution mixing.

13. Pharmaceutical composition comprising, as an excipient, a chitosan silicon dioxide coprecipitate composition according to any of claims 1 to 11.

14. Pharmaceutical composition according to claim 13, further comprising one or more of fillers, lubricants, fat absorbent materials, and pH buffering agents.

15. Pharmaceutical composition according to claim 13 comprising, as an active agent, pseudoephedrine HCl.

16. Use of a chitosan silicon dioxide coprecipitate composition according to any of claims 1 to 11, as an excipient for manufacturing of a sustained release formulation.

17. Use of a chitosan silicon dioxide coprecipitate composition according to any of claims 1 to 11, as an excipient for manufacturing of an immediate release formulation

18. Use according to claim 16 or 17, wherein the sustained release formulation is in the form of a tablet or capsule.

## Patentansprüche

1. Chitosan-Siliziumdioxid-Co-Präzipitat-Zusammensetzung.

2. Zusammensetzung nach Anspruch 1, **dadurch gekennzeichnet, daß** der Gehalt an Siliziumdioxid im Bereich von 1 bis 75 Gew.-% liegt.

3. Zusammensetzung nach Anspruch 2, **dadurch gekennzeichnet, daß** der Gehalt an Siliziumdioxid etwa 50 Gew.-% beträgt.

4. Zusammensetzung nach einem der vorangehenden Ansprüche, **dadurch gekennzeichnet, daß** das Siliziumdioxid in kristalliner oder amorpher Form oder als eine modifizierte Form oder ein Salz von Siliziumdioxid, wie etwa Silikat, vorliegt.

5. Zusammensetzung nach Anspruch 4, **dadurch gekennzeichnet, daß** das Siliziumdioxid in der Form von kolloidalem Siliziumdioxid vorliegt.

6. Zusammensetzung nach einem der vorangehenden Ansprüche, **dadurch gekennzeichnet, daß** das Chitosan-Material einen durchschnittlichen Deacetylierungsgrad von mehr als 50% besitzt.

7. Zusammensetzung nach Anspruch 6, **dadurch gekennzeichnet, daß** das Chitosan-Material einen durchschnittlichen Deacetylierungsgrad von mehr als 75% besitzt.

8. Zusammensetzung nach Anspruch 7, **dadurch gekennzeichnet, daß** das Chitosan-Material ein durchschnittlichen Deacetylierungsgrad von mehr als 85% besitzt.

9. Zusammensetzung nach einem der vorangehenden Ansprüche, **dadurch gekennzeichnet, daß** das Chitosan-Material ein durchschnittliches Molekulargewicht im Bereich von 1.000 bis 10.000.000 g/mol besitzt.

10. Zusammensetzung nach Anspruch 9, **dadurch gekennzeichnet, daß** das Chitosan-Material ein durchschnittliches Molekulargewicht im Bereich von 2.000 bis 100.000 g/mol besitzt.

11. Zusammensetzung nach einem der vorangehenden Ansprüche, **dadurch gekennzeichnet, daß** das Chitosan-Material ein Chitosansalz mit einer organischen oder anorganischen Säure ist.

12. Verfahren zur Herstellung einer Chitosan-Siliziumdioxid-Co-Präzipitat-Zusammensetzung nach einem der Ansprüche 1 bis 11, **dadurch gekennzeichnet, daß** das Co-Präzipitations-Verfahren ausgewählt ist aus Sprühtrocknung, Gefriertrocknung oder Lösungsmischung.

13. Pharmazeutische Zusammensetzung, die, als einen Hilfsstoff, eine Chitosan-Siliziumdioxid-Co-Präzipitat-Zusammensetzung nach einem der Ansprüche 1 bis 11 umfaßt.

14. Pharmazeutische Zusammensetzung nach Anspruch 13, die weiter eines oder mehrere aus der Gruppe Füllstoffe, Gleitmittel, fettabsorbierende Materialien und pHpuffemde Mittel umfaßt.

15. Pharmazeutische Zusammensetzung nach Anspruch 13, die, als einen Wirkstoff, Pseudoephedrin-HCl umfasst.

16. Verwendung einer Chitosan-Siliziumdioxid-Co-Präzipitat-Zusammensetzung nach einem der Ansprüche 1 bis 11 als einen Hilfsstoff für die Herstellung einer Formulierung mit verzögerter Freisetzung.

17. Verwendung einer Chitosan-Siliziumdioxid-Co-Präzipitat-Zusammensetzung nach einem der Ansprüche 1 bis 11 als einen Hilfsstoff für die Herstellung einer Formulierung mit sofortiger Freisetzung.

18. Verwendung nach Anspruch 16, **dadurch gekennzeichnet, daß** die Formulierung mit verzögerter Freisetzung in Form einer Tablette oder Kapsel vorliegt.

## Revendications

1. Composition à base d'un coprécipité de chitosane et de dioxyde de silicium.

2. Composition selon la revendication 1, dans laquelle la teneur en dioxyde de silicium se trouve dans la gamme allant de 1 à 75 % m/m.

3. Composition selon la revendication 2, dans laquelle la teneur en dioxyde de silicium est d'environ 50 % m/m.

4. Composition selon l'une quelconque des revendications précédentes, dans laquelle le dioxyde de silicium est présent sous forme cristalline ou amorphe ou sous une forme modifiée ou sous la forme d'un sel de dioxyde de silicium tel que le silicate.

5. Composition selon la revendication 4, dans laquelle le dioxyde de silicium est présent sous la forme de dioxyde de silicium colloïdal.

6. Composition selon l'une quelconque des revendications précédentes, dans laquelle le chitosane présente un degré moyen de désacétylation supérieur à 50 %.

7. Composition selon la revendication 6, dans laquelle le chitosane présente un degré moyen de désacétylation supérieur à 75 %.

8. Composition selon la revendication 7, dans lequel le chitosane présente un degré moyen de désacétylation supérieur à 85 %.

9. Composition selon l'une quelconque des revendications précédentes, dans laquelle le chitosane possède une masse moléculaire moyenne se trouvant dans la gamme allant de 1000 à 10 000 000 g/mol.

10. Composition selon la revendication 9, dans laquelle le chitosane possède une masse moléculaire moyenne se trouvant dans la gamme allant de 2000 à 100 000 g/mol.

11. Composition selon l'une quelconque des revendications précédentes, dans laquelle le chitosane est un sel de chitosane avec un acide organique ou inorganique.

12. Procédé de production d'une composition à base d'un coprécipité de chitosane et de dioxyde de silicium selon l'une quelconque des revendications 1 à 11, dans lequel le procédé de coprécipitation est choisi parmi le séchage par atomisation, la lyophilisation ou le mélange de solutions.

13. Composition pharmaceutique comprenant, comme excipient, une composition à base d'un coprécipité de chitosane et de dioxyde de silicium selon l'une quelconque des revendications 1 à 11.

14. Composition pharmaceutique selon la revendication 13, comprenant en outre une ou plusieurs charges, un ou plusieurs lubrifiants, matériaux absorbant les graisses, et agents tampons du pH.

15. Composition pharmaceutique selon la revendication 13 comprenant, comme agent actif, du chlorhydrate de pseudoéphédrine.

16. Utilisation d'une composition à base d'un coprécipité de chitosane et de dioxyde de silicium selon l'une quelconque des revendications 1 à 11 comme excipient pour la fabrication d'une préparation à libération prolongée.

17. Utilisation d'une composition à base d'un coprécipité de chitosane et de dioxyde de silicium selon l'une quelconque des revendications 1 à 11 comme excipient pour la fabrication d'une préparation à libération immédiate.

18. Utilisation selon la revendication 16, dans laquelle la préparation à libération prolongée se présente sous la forme d'un comprimé ou d'une capsule.
